(19) Europäisches Patentamt
European Patent Office
Office européen des brevets

(11) Numéro de publication: **0 362 077**
**A1**

(12) **DEMANDE DE BREVET EUROPEEN**

(21) Numéro de dépôt: **89402691.3**

(51) Int. Cl.5: **G01N 33/86**

(22) Date de dépôt: **29.09.89**

(30) Priorité: **29.09.88 FR 8812765**

(43) Date de publication de la demande:
**04.04.90 Bulletin 90/14**

(84) Etats contractants désignés:
**AT BE CH DE ES GB GR IT LI LU NL SE**

(71) Demandeur: **SANOFI**
**40, Avenue George V**
**F-75008 Paris(FR)**

(72) Inventeur: **Lormeau, Jean-Claude**
**34, rue du 8 mai**
**F-76150 Maromme(FR)**

(74) Mandataire: **Peaucelle, Chantal et al**
**S.C. Ernest Gutmann - Yves Plasseraud 67, boulevard Haussmann**
**F-75008 Paris(FR)**

(54) **Procédé de dosage de polyosides anioniques sulfates, et coffret pour la mise en oeuvre de ce procédé.**

(57) Procédé de dosage de polyosides anioniques sulfatés fortement anioniques dans un liquide, basé sur le dosage, grâce à un conjugué anionique révélateur, des sites cationiques restés libres sur une surface revoucerte d'un polycation, et coffret pour la mise en oeuvre de ce procédé.

EP 0 362 077 A1

# PROCEDE DE DOSAGE DE POLYOSIDES ANIONIQUES SULFATES, ET COFFRET POUR LA MISE EN OEUVRE DE CE PROCEDE

L'invention concerne un nouveau procédé de dosage de polyosides anioniques sulfatés, utilisant dans le principe de dosage la structure chimique de ces composés, et non pas leurs activités biologiques. L'invention concerne également un coffret prêt à l'emploi pour la mise en oeuvre de ce procédé.

Les polyosides anioniques sulfatés, tels que les glycosaminoglycanes et notamment l'héparine, dont l'activité anticoagulante est bien connue, sont dosés selon des méthodes basées sur leurs activités biologiques, en particulier leur activité sur divers facteur de la coagulation. C'est ainsi que l'on dose l'héparine par son activité sur le facteur X activé ou le facteur II activé ou encore selon des méthodes plus globales basées sur le temps de coagulation, tels que le temps de Céphaline Kaolin ou TCK ou encore la méthode décrite dans la Pharmacopée Française.

Or, les récents travaux sur l'héparine ont montré qu'il s'agissait d'un produit très hétérogène, aussi bien en ce qui concerne la longueur de ses chaînes que leur taux de charge, et qu'il était possible de les fractionner et de les fragmenter en vue de mettre au point des produits à activité plus spécifique. On a également pu mettre en évidence que l'héparine, outre ses activités bien connues sur la coagulation, intervenait comme régulateur dans divers systèmes biologiques et que des fragments dépourvus d'activité sur la coagulation conservaient néanmoins leur activité régulatrice. Il devenait donc nécessaire de mettre au point une nouvelle méthode de dosage permettant d'évaluer la quantité de produit contenu dans un liquide, et en particulier dans les milieux biologiques, sans avoir recours à une activité biologique.

Dawes, Pepper et al. (Thromb. Haemost., 1985, 54 (3), 630-634) ont proposé une technique qui consiste en un déplacement de l'héparine radioactive, retenue sur une colonne de type Agarose-polycation, par le polyoside anionique sulfaté à tester. Cette méthode est basée sur le principe général du déplacement d'un produit marqué de ses sites de liaisons, par son équivalent non marqué. Les méthodes faisant appel à ce principe présentent l'inconvenient de nécessiter la manipulation de radio-éléments qui à la fois sont onéreux et impliquent des précautions d'emploi.

L'invention se propose de remédier à ces inconvenients et propose un nouveau procédé qui permet le dosage quantitatif des polyosides sulfatés contenus dans un liquide, notamment dans un milieu biologique, en particulier le plasma. Ce dosage est basé sur le caractère anionique fort de ce type de produits et permet le dosage de tout polyoside sulfaté, quelle que soit sa structure ou son type d'activité, sous réserve qu'il ait une quantité de charges anioniques totale d'au moins deux, de préférence d'au moins trois, par unité disaccharidique. Des produits de ce type sont représentés par l'héparine et ses fractions, fragments et dérivés, le pentosane polysulfate, le dextrane sulfate et certains héparanes sulfates; en revanche, l'acide hyaluronique, le dermatane sulfate, les chondroitines sulfates et les héparanes faiblement sulfatés, ne peuvent être dosés par ce procédé.

Le nouveau procédé de l'invention est basé sur le dosage, grâce à un conjugué anionique révélateur, des sites cationiques restés libres sur une surface recouverte d'un polycation, après que celle-ci ait été mise en contact avec le liquide contenant le polyoside anionique sulfaté à doser, le conjugué révélateur étant choisi de telle manière que sa densité de charge lui permette de se fixer sur les sites restés vacants sur le polycation recouvrant la surface, sans toutefois déplacer le polyoside à doser qui s'y trouve préalablement fixé, et la quantité du produit à doser étant déterminée par référence à une courbe d'étalonnage établie simultanément sur la même plaque.

Ce procédé peut être avantageusement mis en oeuvre selon les étapes successives suivantes:

(a) on met en contact une quantité déterminée du liquide contenant le produit à doser avec une surface recouverte d'un produit cationique;

(b) on met en contact l'ensemble ainsi obtenu avec un excès d'un conjugué anionique révélateur, choisi de telle manière que sa densité de charge lui permette de se fixer sur les sites restés vacants sur le produit cationique de l'étape (a) sans toutefois déplacer le polyoside à doser qui s'y trouve préalablement fixé;

(c) on élimine l'excès du conjugué révélateur qui ne s'est pas fixé par liaison ionique avec l'excès du produit cationique de l'étape (a);

(d) on dose, par une méthode appropriée, fonction du conjugué révélateur choisi, la quantité dudit conjugué révélateur qui s'est fixée par liaison ionique au cours de l'étape (b);

(e) on détermine la quantité totale du produit à doser se trouvant dans l'échantillon en rapportant la quantité du conjugué révélateur définie selon la méthode appropriée de l'étape (d) à une courbe d'étalonnage établie simultanément sur la même plaque, selon la même méthode, avec des quantités données du produit que l'on veut doser.

Le résultat est lu sur une courbe d'étalonnage qui indique directement la quantité de polyoside anionique sulfaté par millilitre du milieu biologique.

Selon le procédé de l'invention, le polycation utilisé est un polymère basique choisi dans le groupe constitué par le sulfate de protamine, le polybrène, ou un polyaminoacide tel que la poly-arginine ou la poly-L-lysine. On utilise de préférence une poly-L-lysine dont le poids moléculaire moyen se situe entre 6000 et 9000 daltons. Ce polycation présente en effet l'avantage de donner une très bonne reproductibilité dans les dosages.

D'une manière avantageuse, le conjugué anionique révélateur est constitué d'un glycosaminoglycane lié de façon covalente à un polypeptide, de préférence un enzyme. Il pourrait également être lié à une molécule organique contenant des noyaux aromatiques susceptibles d'être marqués par exemple à l'iode ou au phosphore, par exemple de la tyrosine marquée à l'iode 125.

Dans la suite du texte, nous utiliserons l'abréviation GAG pour le glycosaminoglycane et le terme GAG-P pour tout glycosaminoglycane-polypeptide, chaque conjugué étant désigné par le glycosaminoglycane GAG et le polypeptide P concernés, par exemple GAG-enzyme, GAG-peroxydase, GAG-phosphatase, GAG-albumine, Héparine-P, Héparine-enzyme, Héparine-peroxydase, Héparine-phosphatase, Oligosaccharide-CHO-peroxydase (OS-CHO-peroxydase) et ainsi de suite.

Le GAG utilisé pour la préparation du conjugué doit être suffisamment anionique pour aller se fixer sur les sites laissés vacants, mais ne doit pas être plus anionique que le produit à doser, dont il prendrait alors la place. Le choix du GAG à utiliser pour la préparation du conjugué est donc à effectuer en fonction de la densité de charge du polyoside anionique sulfaté que l'on veut doser.

Le GAG utilisé pour la préparation du conjugué révélateur GAG-P peut être n'importe quel GAG ou l'une de ses fractions ou fragments. Le GAG utilisé est de préférence l'héparine ou l'un de ses fragments. Ceux-ci ont en effet la capacité de se fixer, par leur charges anioniques, aux sites laissés vacants sur le polycation après que le produit à tester ait été mis en contact avec ce dernier. Un mélange de GAG particulièrement approprié pour la préparation du GAG-P est représenté par un mélange de fragments d'héparine constitué de chaînes possédant essentiellement de 4 à 10 unités disaccharidiques.

D'une manière avantageuse, on utilise comme polypeptide pour la préparation du conjugué révélateur GAG-P, un enzyme; celui-ci est choisi parmi ceux qui sont facilement préparables et stables et qui peuvent être dosés aisément, par exemple par action d'un substrat et libération d'un produit coloré que l'on dose par densité optique; la peroxydase de raifort et la phosphatase sont des exemples de tels produits.

Dans un mode particulièrement avantageux de l'invention, le conjugué GAG-enzyme est dilué avec un autre conjugué GAG-P, de préférence avec un GAG-albumine, le GAG étant identique pour les deux conjugués. En effet, la précision du dosage est inversement proportionnelle à l'activité spécifique du GAG-enzyme et, si cette dernière est trop importante, il est difficile de définir des conditions opératoires donnant une précision satisfaisante. On a donc recours de préférence pour pouvoir doser avec une précision de l'ordre du µg/ml à un mélange de conjugués, l'activité spécifique finale du mélange étant établie de manière à avoir une bonne précision de dosage dans les conditions opératoires.

De préférence, le mélange GAG-enzyme et GAG-albumine a une actitivé enzymatique qui se situe dans nos conditions opératoires entre 1 u.DO et 2 u.DO, l'activité spécifique la plus favorable étant de 1,5 u.DO.

De manière avantageuse, on a pu constater que le conjugué formé d'un GAG représenté par un fragment d'héparine préparé par dépolymérisation à l'acide nitreux et possédant un poids moléculaire moyen de 2.000 daltons, notamment le produit désigné OS 4-10-CHO dont la préparation est décrite dans le paragraphe "PREPARATION I a)" ci-dessous, d'une part, et à titre d'enzyme la peroxydase de raifort, constituait un révélateur de choix pour doser dans les milieux biologiques différents polyosides anioniques sulfatés, tels que l'héparine et ses fragments comme ceux vendus sous le nom de Fraxiparine (R)(Choay, France), Fragmin (R) (Kabi, Suède), Lovenox (R)(Pharmuka, France), ou encore tel que le pentosane polysulfate vendu sous la dénomination d'Hémoclar (R)(Midy, France), ou le dextrane sulfate et ses dérivés, ou encore les dextranes solubles fonctionnalisés décrits dans le demande de brevet EP 146 455, ou les GAG hypersulfatés décrits dans le demande de brevet EP 214 879.

Dans un mode particulièrement avantageux de l'invention au cours de l'étape (a) le polycation est adsorbé sur les puits d'une plaque de microtitration en milieu basique, par incubation de 24 à 72 heures, de préférence 48 heures, à une température se situant entre 0° et +20° C, de préférence +4° C.

De façon avantageuse, le conjugué anionique révélateur de l'étape (b) est constitué d'un polyoside anionique lié de façon covalente à un produit choisi dans le groupe constitué par un enzyme et une molécule organique contenant des noyaux aromatiques susceptibles d'être marqués à l'iode ou au phosphore. De préférence, on utilise un enzyme qui évite de mettre en oeuvre des produits marqués.

Dans un autre mode avantageux de l'invention, au cours de l'étape (b) le mélange est incubé au moins 2 heures à une température se situant entre 0° et +20° C, de préférence +4° C.

Selon un mode préféré de l'invention, le conjugué anionique révélateur de l'étape (b) est constitué d'un polyoside anionique, notamment un GAG, lié de façon covalente à un enzyme et au cours de l'étape (d) on utilise une méthode de dosage enzymatique, l'enzyme du conjugué révélateur étant dosable par exemple par action sur un substrat et libération d'un produit coloré que l'on dose par densité optique.

Les différents lavages sont effectués à l'aide de soluté chloruré isotonique (NaCl 9 P. 1000).

D'une manière avantageuse, le milieu basique utilisé pour la mise en solution du polycation est un tampon tris-phosphate ou un tampon bicarbonate à un pH compris entre 9 et 10; on utilise de préférence un tampon bicarbonate d'une force ionique comprise entre 0,05 M et 0,2 M, en particulier 0,1 M, pH 9,6. Le polycation est mis en solution dans le milieu basique à la concentration de 100 µg/ml et l'on dépose dans chaque puits 250 µg de cette solution que l'on laisse incuber pendant 48 heures.

Le dosage sera d'autant plus sensible que la concentration en polycation sera plus faible, mais la gamme de réponse sera d'autant plus large que la concentration sera plus élevée, la sensibilité étant inversement proportionnelle à la concentration en polycation. D'une manière avantageuse, on utilise une concentration de 100 µg/ml comme indiqué ci-dessus pour doser un GAG présent dans le plasma à une concentration comprise entre 0,1 et 0,5 µg/ml. Les résultats obtenus sont particulièrement satisfaisants dans les conditions indiquées ci-dessus lorsque le polyoside anionique sulfaté à doser est un fragment régulier d'héparine, c'est-à-dire un fragment constitué de disaccharides trisulfatés, et dont le poids moléculaire moyen se situe entre 5.000 et 6.000 daltons.

De façon avantageuse, pour la réalisation du procédé de l'invention, on dépose dans chaque puits, après lavage pour élimination du polycation résiduel, 200 µl du liquide contenant le polyanion sulfaté à doser, ce dernier ainsi que le liquide utilisé pour la réalisation de la courbe d'étalonnage étant additionné d'un inhibiteur de protéase qui permet à la fois de respecter le polycation et, lorsque le liquide est un milieu biologique, en particulier du plasma, d'empêcher la formation de thrombine et donc d'un caillot qui perturberait le dosage. On utilise de préférence comme inhibiteur de protéase la benzamidine que l'on additionne à une concentration se situant entre 0,0025 M et 0,1 M, de préférence 0,0125 M, et on laisse l'incubation s'effectuer pendant au moins 2 heures entre 0° et +20° C, de préférence +4° C.

Avantageusement, lorsque le mélange de conjugués révélateur utilise comme enzyme la peroxydase, le mélange comporte des quantités de GAG-enzyme et de GAG-albumine exprimées en équivalent de produit pur de l'ordre de 3,4 µg/ml de peroxydase et 7,7 µg/ml d'albumine. Dans ces conditions on utilise de préférence 200 µl par puits du mélange révélateur.

Dans un mode de réalisation avantageux du procédé de l'invention, lorsque le conjugué révélateur est un GAG-peroxydase, le substrat utilisé pour révéler la quantité d'enzyme fixée dans les puits est constitué d'un mélange d'orthophénylène diamine et de peroxyde d'hydrogène. On utilise par exemple pour chaque puits 100 µl d'un mélange contenant 0,5 mg d'orthophénylène diamine dans un ml de tampon citrate de sodium/ HCl 0,1 M, pH 3,5 et 0,5 µl de peroxyde d'hydrogène. Dans ces conditions, on laisse incuber environ 10 minutes à température ambiante (environ 20 à 25° C), puis on arrête la réaction par addition d'acide sulfurique 1N, avantageusement 100 µl par puits.

Dans un mode particulièrement avantageux de l'invention, lorsque le conjugué révélateur est un GAG-peroxydase et le substrat de dosage l'orthophénylène diamine additionnée de peroxyde d'hydrogène, la lecture de la densité optique s'effectue à 492 nm, soit directement au fond du puits, soit en transférant dans une cuve de spectrophotomètre.

On obtient la courbe d'étalonnage de la figure 1 qui donne en ordonnée l'adsorption à 492 nm et en abscisse le log de la concentration en µg/ml. La lecture de la densité optique de l'échantillon à doser permet, en se référant à la courbe d'étalonnage, d'en déduire la concentration du produit à doser dans un ml du milieu biologique.

Dans un autre mode de réalisation, le conjugué révélateur est un GAG-phosphatase, et le substrat de dosage le 4-nitrophénylphosphate, la lecture de la densité optique s'effectuant à 405 nm.

L'invention concerne également un coffret destiné à la mise en oeuvre du procédé selon l'invention. Ce coffret contient:

- Plaque de microtitration,
- Polycation,
- Conjugué révélateur constitué par un polyanion conjugué à un révélateur choisi dans le groupe constitué par un enzyme et une molécule organique susceptible d'être marquée,
- Substrat de dosage choisi en fonction du conjugué révélateur.

Dans un aspect particulier de l'invention le coffret contient:

- Plaque de microtitration,
- Polycation choisi, dans le groupe constitué par le sulfate de protamine, le polybrène, la poly-arginine et la poly-L-lysine,

- GAG-enzyme lyophilisé,
- Substrat de l'enzyme choisi, générateur d'un colorant.

Dans un mode particulièrement avantageux de l'invention le coffret comporte:

- Plaque de microtitration,
- Polycation,
- GAG-enzyme lyophilisé, l'enzyme étant choisi dans le groupe constitué par la peroxydase de raifort et la phosphatase,
- GAG-albumine lyophilisé,
- Substrat de l'enzyme choisi, générateur d'un colorant.

Dans un autre mode particulier de l'invention le coffret comporte un multiple de chacun des élements suivants:

- Plaque de microtitration : 1,
- Poly-L-lysine : 25 ml à 100 µg/ml,
- GAG-peroxydase de raifort lyophilisé : 68 µg,
- GAG-albumine : 54 µg,
- Orthophénylène diamine : 10 ml (5 mg).

De préférence le coffret selon la présente invention est accompagné d'une méthode d'utilisation comme définie ci-dessus.

Dans un autre mode avantageux de l'invention, les plaques de microtitration sont recouvertes de polycation et prêtes à l'emploi.

Les conjugués GAG-P, notamment GAG-enzyme et GAG-albumine, appartiennent à la famille des N-polyosides-polypeptides de formule I suivante:

$(Oside - Z' - CH_2 - NH)_nP'$ (I)

dans laquelle:

- P' est le radical n-valent d'un polypeptide P lié aux n groupes NH provenant de n groupes amino libres présents à l'origine dans la molécule de P;
- Z' est l'unité saccharidique terminale réductrice du polysaccharide Oside-Z dans sa forme aldéhydique, soit naturelle, soit provenant de la dépolymérisation nitreuse, privée du group aldéhydique de Z;
- Oside est le reste du polysaccharide Oside-Z';
- n est un nombre entier de 3 à 30;

ou, le cas échéant, un de ses sels pharmaceutiquement acceptables.

Il est en effet possible d'effectuer un greffage de la chaîne polyosidique sur la chaîne peptidique en un point unique de ladite chaîne polyosidique en ayant recours à un groupe aldéhydique unique présent ou généré à l'extrémité de la chaîne polyosidique.

Il est bien connu que les sucres en général et les polysaccharides en particulier sont caractérisés par une structure hémiacétalique (cyclique ou fermée) en équilibre avec la structure aldéhydique correspondante (linéaire ou ouverte) selon le Schéma (A)

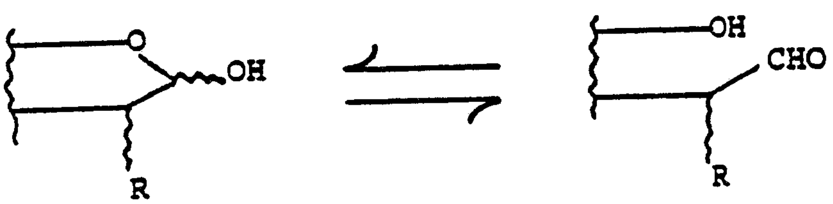

(A)

R étant par exemple un hydroxyle ou un groupe amino libre ou sulfaté, ledit équilibre étant, dans les sucres naturels, presque totalement déplacé vers la gauche.

Il est en outr· ·onnu que certaines méthodes de dépolymérisation de polysaccharides, notamment la dépolymérisation par action de l'acide nitreux, ci-après désignée "dépolymérisation nitreuse", conduisent à la transformation des unités osidiques. Ainsi, par exemple, les unités glycosidiques de l'héparine sont transformées, par la dépolymérisation nitreuse, en unités 2,5-anhydromannosidiques selon le Schéma (B)

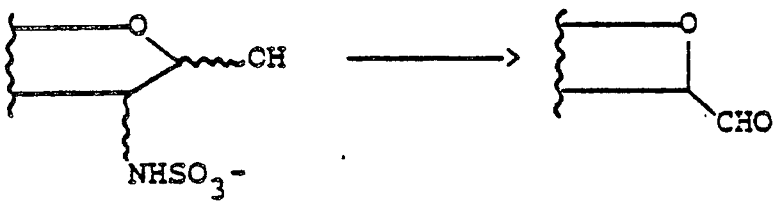

(B)

ladite unité 2,5-anhydromannosidique présentant un groupe aldéhydique.

La technique de dépolymérisation nitreuse a permis de préparer des héparines de bas poids moléculaire ayant à l'extrémité de la majorité des espèces le groupement, 2,5-anhydromannose, donc un groupe aldéhydique.

On a maintenant trouvé qu'en faisant réagir un polysaccharide avec un polypeptide, la forme aldéhydique dudit polysaccharide réagit avec des amines libres dudit polypeptide pour donner une poly-base de Schiff. Cette réaction comporte le déplacement de l'équilibre du Schéma (A) ci-dessus vers la droite et la formation de la poly-base de Schiff.

On a aussi trouvé que la même réaction a lieu avec des polysaccharides obtenus par dépolymérisation nitreuse selon le Schéma (B) ci-dessus, et que la poly-base de Schiff se forme rapidement avec des rendements satisfaisants.

De plus, comme les polyosides n'ont qu'une seule extrémité réductrice, le couplage ne peut avoir lieu qu'au niveau de cette extrémité et la structure tertiaire du polypeptide est ainsi conservée.

On a ultérieurement trouvé qu'en faisant réagir le polysaccharide avec le polypeptide en présence d'un agent réducteur, on obtient directement le conjugué polysaccharide/polypeptide sans isoler la poly-base de Schiff intermédiaire, avec de très bons rendements.

Dans la présente description le polypeptide, désigné "P", est schématiquement représenté par la formule II suivante:

$P^{\circ}(NH_2)_m$ (II)

dans laquelle $P^{\circ}$ est le radical m-valent du polypeptide P auquel sont liés ses m groupes fonctionnels $NH_2$ libres, lesdites m groupes fonctionnels $NH_2$ étant le groupe amino terminal et les (m-1) groupes amino en position epsilon des lysines contenues dans le polypeptide.

Dans la formule (II) ci-dessus, m représente la totalité des groupes amino disponibles pour la formation des poly-bases de Schiff, mais le couplage n'a lieu que sur un certain nombre, désigné "n", de ces groupes amino; le radical n-valent lié aux groupes amino qui ont réagi avec le groupe aldéhydique de Z est ci-après désigné $P'$.

Il est entendu que, dans la présente description, le concepte élargi de "valence" se réfère aux liaisons libres des radicaux ou des structures concernés. Ainsi, le radical $P'$ m-valent ou n-valent est le résidu du polypeptide P avec ses m ou n liaisons libres telles qu'illustrées ci-dessus et les structures bivalentes Ia″, Ib″, Ic″ et Id″ ci-dessous s'expliquent toutes seules.

Dans la présente description, le polysaccharide est schématiquement représenté par la formule

Oside-Z (III)

Z étant l'unité saccharidique terminale réductrice dudit polysaccharide dans sa forme aldéhydique soit naturelle, soit provenant d'une dépolymérisation nitreuse et Oside étant le reste de la chaîne dudit polysaccharide.

Le radical monovalent Oside-Z′- représente le polysaccharide Oside-Z tel que défini ci-dessus, privé du groupe aldéhydique de Z.

Plus particulièrement, dans la formule (I) Oside- représente le radical d'un polysaccharide Oside-Z (III), appelé également "polyoside", provenant de substances naturelles biocompatibles, sans résidus aglycones, ledit polyoside étant privé d'une unité saccharidique terminale réductrice (Z).

Le terme "biocompatible" tel qu'utilisé dans le présent descriptif désigne la propriété de polysaccharides naturels, ayant un poids moléculaire de préférence inférieur à 100.000, notamment compris entre 1.200 et 50.000, et de leurs dérivés ou analogues dont les constituants se trouvent naturellement dans l'organisme des mammifères et qui, de ce fait, peuvent être facilement métabolisés et éliminés.

De façon avantageuse, le polyoside provenant de substances naturelles biocompatibles est un glycosaminoglycane tel que défini par R.W. Jeanloz, Arthritis and Rheumatism, 1960, 3, 233-237 et par B. Casu, Advances in Carbohydrate Chemistry and Biochemistry, 1985, 43, 51-134.

De façon préférentielle, dans la formule I ci-dessus, Oside représente le radical d'un glycosaminoglycane Oside-Z choisi parmi l'héparine, les fragments d'héparine, la chondroitine sulfate 4-S, la chondroitine sulfate 6-S, l'héparane sulfate et le dermatane sulfate, privé de l'unité terminale réductrice Z.

Dans un aspect particulier et avantageux de l'invention, le N-polyosyl-polypeptide provient d'un glycosaminoglycane du type héparinique Oside-Z dont le motif Z représente le motif terminal réducteur d'une chaîne d'héparine ayant subi une dépolymérisation nitreuse. Il est représenté par la formule (Ia) suivante:

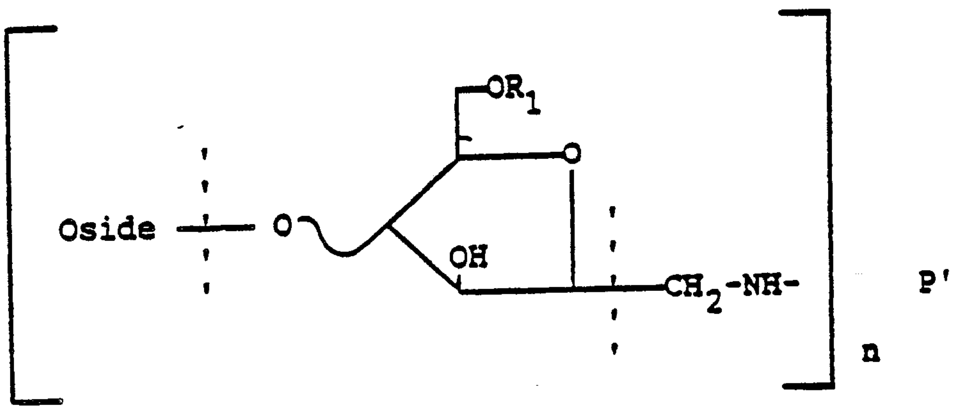

(Ia)

qui correspond à la formule (I), le groupement entre les lignes en pointillé étant le radical Z′ et $R_1$ étant H ou $SO_3^-$.

Dans la formule (Ia) ci-dessus, Oside désigne, selon un aspect préférentiel de la présente invention, le radical provenant d'une héparine, représenté par la formule

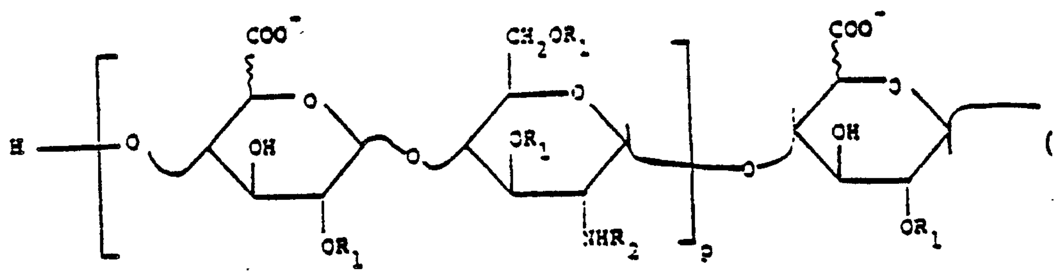

(Ia′)

dans laquelle $R_1$ représente l'hydrogène ou un groupe $SO_3^-$, $R_2$ représente un groupe acétyle ou un groupe $SO_3^-$ et p est de 4 à 24.

Il est entendu que la valeur de p, dans le radical (Ia′), est telle qu'elle représente le nombre d'unités disaccharidiques de fragments d'héparine provenant d'une dépolymérisation nitreuse de l'héparine naturelle, dans des mélanges ayant avantageusement une masse moléculaire moyenne se situant entre 2000 et 8000 daltons, avantageusement entre 2000 et 6500 daltons, de préférence d'environ 2000 à 2500 daltons.

Dans un autre aspect particulier de l'invention, le N-polyosyl-polypeptide provient d'un polysaccharide Oside-Z dont le motif Z représente le motif terminal réducteur naturel d'un glycosaminoglycane de type héparinique. Il est représenté par la formule (Ib) suivante:

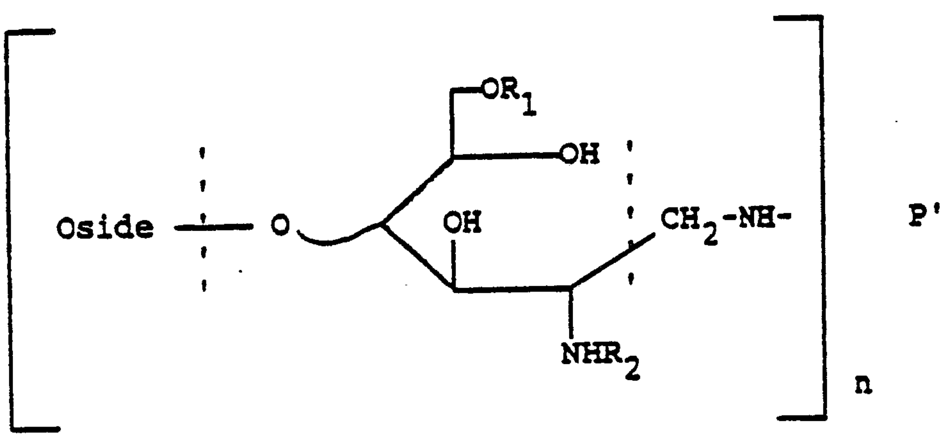

(Ib)

qui correspond à la formule (I), le groupement entre les lignes en pointillé étant le radical Z′, $R_1$ étant H ou $SO_3^-$, $R_2$ étant H, $SO_3^-$ ou acétyle et Oside étant le reste de la chaîne héparinique.

Dans la formule (Ib) ci-dessus, $R_2$ représente de préférence $SO_3^-$ ou acétyle et Oside représente de préférence le radical (Ia′), dans lequel p est de 10 à 100, la valeur de p étant telle qu'elle représente le nombre d'unités disaccharidiques qui forment les mélanges de chaînes présentes dans l'héparine naturelle.

Dans un autre aspect particulier de l'invention, le N-polyosyl-polypeptide provient d'un polysaccharide Oside-Z dont le motif Z représente le motif terminal réducteur naturel d'un glycosaminoglycane de type chondroitine ou dermatane sulfate. Il est représenté par la formule (Ic) suivante:

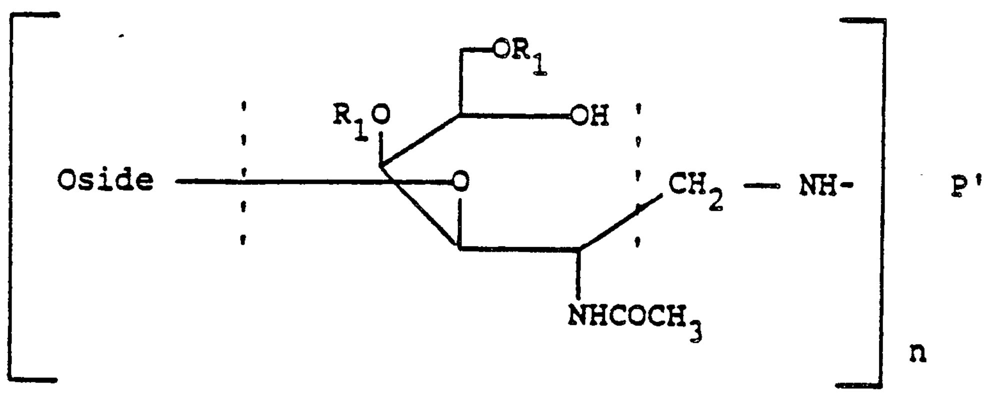

(Ic)

qui correspond à la formule (I), le groupement entre les lignes en pointillé étant le radical Z′, l'un des $R_1$ étant H et l'autre $SO_3^-$ et Oside étant le reste de la chaîne de la chondroitine sulfate ou du dermatane sulfate.

Dans la formule (Ic) ci-dessus, le radical Oside est représenté par la formula (Ic′)

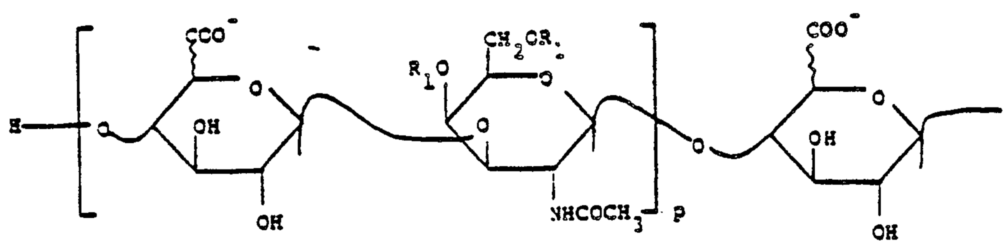

(Ic')

où l'un des $R_1$ représente l'hydrogène et l'autre un groupe $SO_3^-$ et p est de 10 à 100, la valeur de p étant telle qu'elle représente le nombre d'unités disaccharidiques qui forment les mélanges des chaînes présentes dans la chondroitine sulfate ou le dermatane sulfate.

Dans un autre aspect particulier de l'invention, le N-polyosyl-polypeptide provient d'un polysaccharide Oside-Z ayant subi une dépolymérisation dans des conditions telles que la chaîne possède un nombre de motifs impairs dont le motif Z réducteur représente un acide uronique, acide D-glucuronique ou L-iduronique. Il est alors représenté par la formule (Id) suivante:

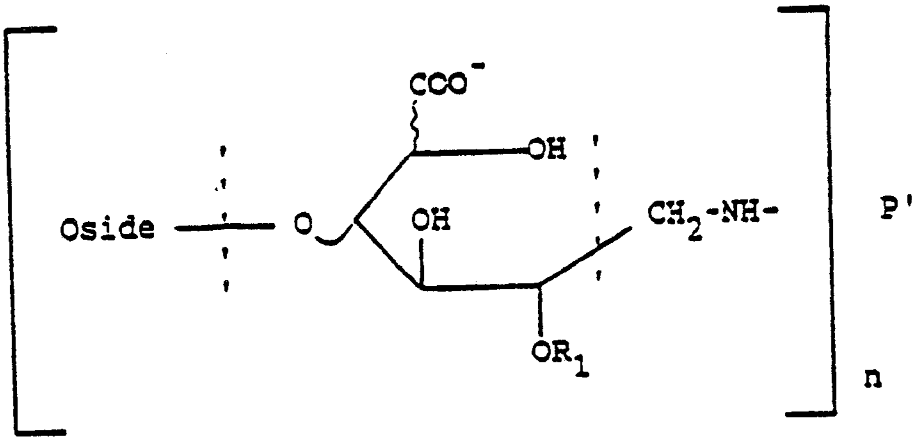

(Id)

qui correspond à la formule (I), le groupement entre les lignes en pointillé étant le radical Z′, $R_1$ étant H ou $SO^-_3$, et Oside étant le reste de la chaîne polysaccharidique.

La présente invention comprend également des composés de formules (Ia) et (Ib) ci-dessus, dans lesquelles Oside représente le reste de fragments d'héparine homogènes au regard de leur masse moléculaire (formula Ia′, p = 1-10) et contenant éventuellement le site de fixation à l'antithrombine III. Elle comprend en outre des composés de formule Ib ci-dessus, dans laquelle Oside représente le reste d'un oligosaccharide de synthèse (formule Ia′, p = 1-6).

Dans les formules (Ia), (Ib), (Ic) et (Id) ci-dessus, le radical Z′ peut être mieux représenté par les structures bivalentes respectives suivantes:

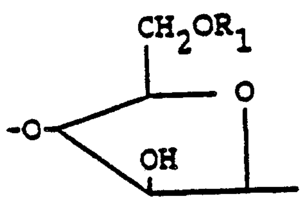

(Ia")

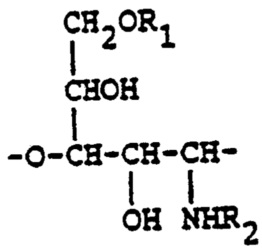

(Ib")

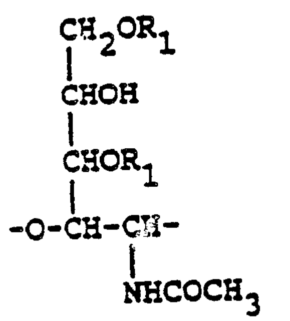

(Ic")

et

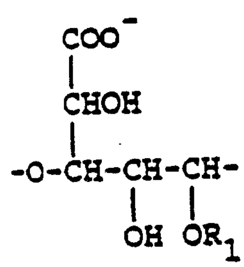

(Id")

dans lesquelles $R_1$ et $R_2$ sont tels que définis ci-dessus et la stéréoconfiguration est celle de l'unité terminale réductrice Z.

Dans la formule I ci-dessus, P′ est avantageusement le radical n-valent d'une protéine enzymatique, en particulier un enzyme capable d'agir sur un substrat en libérant un produit coloré, ce dernier pouvant être dosé par densité optique. La peroxydase de raifort et la phosphatase sont des exemples de tels enzymes. Des résultats très satisfaisants et reproductibles ont été obtenus avec la peroxydase de raifort.

D'une manière avantageuse, le N-polyosyl-polypeptide utilisé à titre de GAG-enzyme pour la mise en oeuvre de la présente invention correspond à la formule (Ia) ci-dessus, dans laquelle P′ est le résidu d'une molécule de peroxydase de raifort et n est égal à 3.

Pour préparer le conjugué GAG-enzyme utile pour la mise en oeuvre de la présente invention, on procède par greffage covalent des motifs amines libres de l'enzyme P sur les chaînes du GAG ne comportant chacune qu'un seul groupement aldéhydique susceptible de réagir avec un motif amine.

Plus particulièrement, le procédé pour la préparation d'un GAG-enzyme de formule (I) ci-dessus est caractérisé en ce qu'on traite l'enzyme P représenté par la formule

$P^{\circ}(NH_2)_m$ (II)

dans laquelle $P^{\circ}$ est le radical m-valent de l'enzyme P auquel sont liés ses m groupes fonctionnels $NH_2$ libres, lesdits m groupes fonctionnels $NH_2$ étant le groupe amino terminal et les groupes amino en position epsilon des (m-1) lysines contenues dans l'enzyme, avec un excès d'un GAG représenté par la formule III suivante:

Oside - Z (III)

Z étant l'unité saccharidique terminale réductrice dudit GAG dans sa forme aldéhydique soit naturelle, soit provenant d'une dépolymérisation nitreuse et Oside étant le reste de la chaîne dudit polysaccharide et on soumet la poly-base de Schiff ainsi obtenue de formule

$(Oside\text{-}Z'\text{-}CH=N\text{-})_nP'$ (IV)

dans laquelle le radical monovalent Oside-Z'- représente le polysaccharide Oside-Z tel que défini ci-dessus, privé du groupe aldéhydique de Z, et P' et n sont tels que définis ci-dessus, à une réduction avec un cyanoborohydrure.

De façon avantageuse, les polyosides naturels biocompatibles Oside-Z utilisés comme produits de départ III sont des glycosaminoglycanes tels que définis ci-dessus (GAGs), notamment des substances constituées de chaînes dans lesquelles alternent des acides uroniques (acide D-glucuronique ou acide L-iduronique) et des sucres aminés, ces derniers pouvant être des glucosamines lorsqu'il s'agit de glycosaminoglycanes de type héparinique, ou des galactosamines lorsqu'il s'agit de glycosaminoglycanes de type chondroitine sulfate, à savoir la chondroitine sulfate 4-S, la chondroitine sulfate 6-S et le dermatane sulfate, les groupements carboxyles des motifs osidiques desdits GAGs pouvant être salifiés ou estérifiés, les groupes hydroxyles étant diversement substitués par des groupements fonctionnels, de préférence des groupes sulfates, et les groupements aminés étant substitués soit par des groupes sulfate, soit par des groupes acétyle.

De tels GAGs sont constitués d'un mélange de chaînes hétérogènes au regard de leurs poids moléculaires, ceux-ci pouvant varier de 1.800 à 50.000 daltons, et au regard de la position et du nombre de leurs groupements fonctionnels.

De préférence, le polyoside de départ III est un glycosaminoglycane choisi parmi l'héparine, les fragments d'héparine, la chondroitine sulfate 4-S, la chondroitine sulfate 6-S, l'héparane sulfate et le dermatane sulfate.

Des composés de départ III particulièrement préférés sont les glycosaminoglycanes de type héparinique, c'est-à-dire l'héparine, l'héparane sulfate et leurs dérivés obtenus soit par fractionnement à partir de l'héparine ou de l'héparane sulfate, soit par hémi-synthèse ou synthèse. De tels polyosides ont été largement décrits dans la littérature. Il peut s'agir de produits naturels obtenus par fractionnement ou synthèse, tels que ceux décrits dans les brevets français 2 440 376 et 2 461 719 ou dans EP 84 999 et 113 599; il peut s'agir également de produits obtenus par dépolymérisation tels que ceux décrits dans les brevets européens 37 319, 40 144 et 181 252. Dans un aspect particulièrement préféré de l'invention, on utilise des mélanges de fragments d'héparine obtenus par dépolymérisation nitreuse ayant une masse moléculaire inférieure à 10.000 daltons, en particulier ceux ayant une masse moléculaire moyenne se situant entre 2.000 et 2.500 daltons. On peut également avantageusement utiliser des fragments d'héparine homogènes au regard de leur masse moléculaire ou des oligosaccharides obtenus par synthèse qui sont homogènes tant en ce qui concerne leur masse moléculaire que la position et le nombre de leurs groupes fonctionnels. Dans la suite du texte, le mélange de chaînes hépariniques obtenues par dépolymérisation nitreuse et ayant un poids moléculaire moyen de 2.000 daltons, préparé comme décrit dans la PREPARATION I a) ci-dessous, sera désigné par la référence "OS 4-10-CHO".

Un autre produit de départ avantageux Oside-Z est le mélange de fragments d'héparine obtenu par dépolymérisation nitreuse et décrit dans EP 37 319; il est désigné "CY 222-CHO".

On peut également choisir comme polyosides des dérivés de l'héparine dépourvus de site de fixation à l'antithrombine III (AT III), soit que les chaînes d'héparines aient été fractionnées de manière à éliminer les chaînes oligosaccharidiques comportant le site de fixation à l'AT III en ayant recours par exemple à une chromatographie d'affinité sur résine Sépharose-AT III ou à des chromatographies d'échange d'ions, comme il est décrit dans E. Sache et al., Thromb. Res., 1982, 25, 443-458, soit que ces sites aient été détruits par exemple par dépolymérisation à l'acide periodique et bêta-élimination exhaustive. On rappellera ici que l'antithrombine III ou AT III est un inhibiteur plasmatique de la coagulation, actif sur différentes

protéases plasmatiques et dont l'activité est fortement accrue par l'héparine agissant comme co-facteur.

D'une manière avantageuse, le couplage entre l'un des motifs aminés de l'enzyme P et la chaîne du GAG Oside-Z s'effectue au niveau du motif terminal de la chaîne Oside-Z, cette dernière ayant ou non subi une opération préalable de fragmentation par dépolymérisation.

Lorsqu'on utilise, comme produit de départ, un GAG Oside-Z dont le motif terminal Z est un groupe 2,5-anhydromannosique obtenu par dépolymérisation nitreuse selon le Schéma (B) ci-dessus, il est préférable que la réaction de couplage soit effectuée sur ledit Oside-Z immédiatement après sa préparation, pour éviter que le groupe aldéhydique, notoirement peu stable, perde sa réactivité.

Dans la mesure où l'on souhaite obtenir une chaîne polyosidique de type héparinique dépourvue de site de fixation à l'antithrombine III, el est avantageux d'utiliser comme produit de départ un GAG ayant subi une dépolymérisation à l'acide periodique, suivie d'une béta-élimination exhaustive, ce procédé permettant de couper préférentiellement la chaîne héparinique entre les carbones en positions 2 et 3 de tous les acides uroniques non sulfatés tel que le GAG décrit par Casu et al., Arzneim. Forsch./Drug Res., 1986, 36 - (1), n. 4, 637-646. Or, on sait qu'un tel motif est présent dans le site de fixation de l'héparine à l'antithrombine III.

La quantité de chaînes polyosidiques à mettre en oeuvre doit être en fort excès par rapport aux chaînes peptidiques dans un rapport de 100 à 2000 moles de Oxide-Z pour 1 mole de la substance peptidique P. Cet excès est avantageusement de 500 à 1500 moles d'Oside-Z pour 1 mole de la substance peptidique P. De préférence, lorsque l'on met en oeuvre de la peroxydase de raifort, on utilise 1000 à 1500 moles de Oside-Z pour 1 mole de peroxidase.

Le milieu réactionnel est un milieu aqueux qui doit être dépourvu d'amines primaires ou secondaires susceptibles d'interférer avec la réaction de couplage entre Oside-Z et le polypeptide P. D'une manière avantageuse, le milieu aqueux est un tampon tel qu'un tampon phosphate ou bicarbonate ou tout autre tampon susceptible d'être ajusté au pH approprié par addition d'un acide minéral ou d'un hydroxyde alcalin. Eventuellement, il peut être additionné d'un solvant organique miscible à l'eau tel que l'éthanol, l'acétone, la diméthylformamide.

Dans un mode préféré de l'invention, on utilise un tampon phosphate 0.05 M additionné de chlorure de sodium. On y adjoint, si nécessaire, du polysorbate 80 (dérivés du sorbitane monooléate ou tween), par exemple à raison de 1 p. 10.000, ce surfactant permettant d'éviter l'adsorption de la substance peptidique P sur les surfaces.

Le pH du milieu réactionnel est choisi de façon à ce que l'amine soit sous forme protonée, il peut varier de 6 à 9, il est toutefois avantageusement de 7 à 8, de préférence voisin de la neutralité.

La température de réaction peut varier en fonction de la substance peptidique utilisée, elle se situe avantageusement entre +2°C et +35°C la réaction étant poursuivie pendant 2 à 15 jours. De préférence, on opère à température basse, par exemple à +4°C, sourtout lorsque la substance peptidique est un enzyme, un grand nombre de ces derniers étant thermolabiles, ce qui est en particulier le cas des enzymes thrombolytiques. Il faut noter toutefois que plus la température est basse, plus la cinétique de la réaction est lente. La durée de la réaction sera donc variable selon la température choisie. De facon avantageuse, on laisse la réaction s'effectuer sous agitation à +4°C pendant 2 à 8 jours si le polyoside utilisé possède un groupement aldéhydique provenant d'une dépolymérisation nitreuse. En particulier lorsque la substance peptidique mise en oeuvre est un enzyme thrombolytique tel que l'urokinase, la pro-urokinase ou l'activateur tissulaire du plasminogène, ou encore lorsqu'il s'agit d'un enzyme tel que la peroxydase ou la phosphatase susceptible d'être dosé facilement par action sur un substrat, le maintien de la réaction à +4°C pendant une durée de 3 à 5 jours permet d'obtenir un rendement de greffage satisfaisant.

La poly-base de Schiff de formule (IV) ainsi obtenue peut être isolée selon les techniques connues, par exemple par précipitation avec un sel approprié, tel que le sulfate d'ammonium, et soumise à une réduction avec un cyanoborohydrure, tel que le cyanoborohydrure de sodium.

La réduction peut également être faite directement dans le milieu réactionnel, sans isoler la poly-base de Schiff.

Il est également possible de passer directement aux composés I par réaction du polypeptide P avec le polyoside Oside-Z en présence de cyanoborohydrure de sodium dans les conditions ci-dessus.

De cette façon, la poly-base de Schiff intermédiaire est réduite au fur et à mesure qu'elle se forme et le produit I est isolé en tant que seul produit final.

Pour séparer le conjugué ainsi obtenu des produits qui n'ont pas réagi, on peut opérer par précipitation, cette opération pouvant s'effectuer par exemple au moyen de sulfate d'ammonium que l'on additionne au milieu réactionnel en quantité suffisante pour saturer la solution, c'est-à-dire à raison d'environ 600 mg/ml. On laisse ensuite décanter et on centrifuge.

Cette opération peut être répetée après reprise du précipité en additionnant à nouveau celui-ci de

sulfate d'ammonium dans les mêmes conditions.

Pour récupérer le N-polyosyl-polypeptide sous forme pure, on effectue une gel perméation à l'aide d'un gel choisi en fonction de la nature et de la taille du polypeptide. La gel filtration s'effectue de manière avantageuse sur une colonne contenant un gel de type ultrogel, par exemple celui vendu par la Société IBF (France) sous le nom d'Ultrogel (R) AcA 44 ou celle vendue par Pharmacia sous le nom de Sephacryl (R) S 200.

Dans un mode de réalisation avantageux, on dissout le précipité obtenu à l'étape précédente dans un tampon approprié à la substance peptidique P mise en oeuvre, on dépose ensuite la solution sur la colonne qui a été préalablement équilibrée à l'aide du même tampon. La gel filtration est suivie au moyen d'un spectrophotomètre à 280 nm et le pic de protéine est récupéré. Il contient la totalité du produit, dont on effectue ensuite la caractérisation et le dosage.

Les poly-bases de Schiff de formule (IV) ci-dessus sont des produits nouveaux qui représentent les intermédiaires clé dans la préparation des N-polyosyl-polypeptides de formule (I). Elles constituent donc un aspect ultérieur de la présente invention.

De façon préférentielle, dans la formule (IV) ci-dessus, Oside représente le radical d'un glycosaminoglycane Oside-Z choisi parmi l'héparine, les fragments d'héparine, la chondroitine sulfate 4-S, la chondroitine sulfate 6-S, l'héparane sulfate et le dermatane sulfate, privé de l'unité terminale réductrice Z.

Particulièrement avantageuses sont les poly-bases de Schiff provenant d'un polysaccharide Oside-Z dont le motif Z représente le motif terminal réducteur d'une chaîne de glycosaminoglycane héparinique ayant subi une dépolymérisation nitreuse. Elle est représentée par la formule (IVa) suivante:

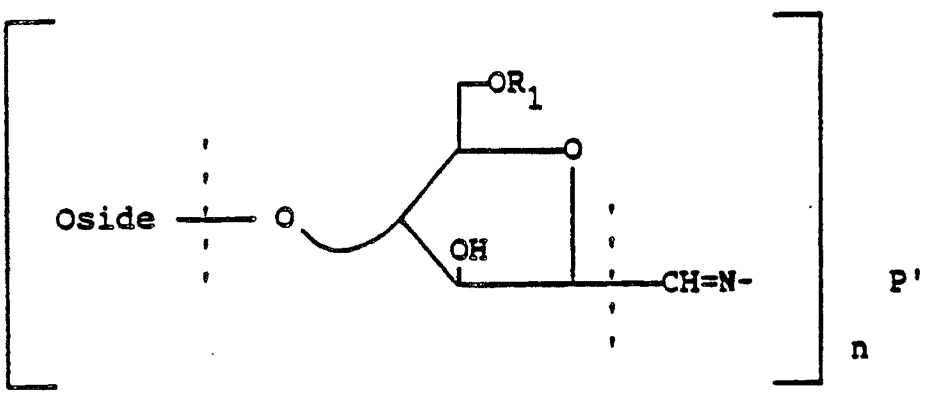

(IVa)

qui correspond à la formule (IV), le groupement entre les lignes en pointillé étant le radical Z′ et $R_1$ étant H ou $SO_3^-$.

D'une manière avantageuse, la poly-base de Schiff de l'invention correspond à la formule (IVa) ci-dessus, dans laquelle P′ est une molécule de péroxydase en particulier la peroxydase de raifort ou la phosphatase, et n est égal ou inférieur à 6, de préférence 3.

La poly-base de Schiff (IVa) ci-dessus est transformée dans le N-polyosyl-polypeptide correspondant (Ia) par réduction avec le cyanoborohydrure de sodium.

L'invention ne se limite pas aux aspects particuliers décrits ci-dessus, elle en englobe au contraire toutes les variantes; elle sera mieux comprise à l'aide des exemples d'application qui vont suivre.

**PREPARATION I**

Préparation du conjugué GAG-enzyme selon la formule (I), dans lequel P est la peroxydase de raifort et Z-oside est le OS 4-10-CHO

La peroxydase de raifort peut être achetée dans le commerce. Il s'agit, par exemple de la peroxydase vendue par SERVA, France. Dans l'exemple qui va suivre, on a utilisé la peroxydase SERVA Lot 18077, dont l'activité est de 1,126 u/mg.

**a)** Génération de la terminaison aldéhydique par dépolymérisation de l'héparine:

Le fragment d'héparine a été préparé de la manière suivante:
500 g d'héparine injectable, sous forme de sel de sodium, sont mis en solution dans 4500 ml d'eau déminéralisée, à une température de 18° C.

Le rapport YW/USP de l'héparine utilisée est voisin de 1, ces titres présentant une valeur de l'ordre de 160-170.

La solution obtenue est mise sous agitation énergique, et son pH est abaissé à 2,5 par addition d'acide chlorhydrique concentré. On ajoute alors 15 g de nitrite de sodium dissous dans 300 ml d'eau. Le pH de la réaction est ajusté à 2,5 par de l'acide chlorhydrique concentré et le volume total de la solution est amené à 5000 ml. On laisse la réaction s'effectuer pendant 45 minutes puis on vérifie l'absence d'ions nitreux résiduels dans la solution réactionnelle, par exemple au moyen de papier indicateur imprégné d'iodure de potassium amidonné (développement d'une coloration bleue-violacée en présence d'ions $NO_2^-$).

On laisse la réaction se poursuivre jusqu'à disparition totale des ions nitreux en absence de réaction au papier iodo-amidonné, en effectuant des contrôles toutes les 3 ou 4 minutes.

Lorsque ces contrôles deviennent négatifs, la réaction est considérée comme étant achevée.

Les produits de la réaction sont récupérés par addition de 10 litres d'éthanol. Après 48 heures de repos, le produit est décanté et on élimine le surnageant.

Le précipité est redissous dans 9 litres d'eau déminéralisée. On ajoute 100 g de chlorure de sodium, et on abaisse le pH de la solution à 3,8 à l'aide d'acide chlorhydrique con- centré. Le volume est ajusté exactement à 10 litres à l'aide d'eau déminéralisée, et l'on ajoute sous agitation énergique 10 litres d'éthanol. On laisse reposer 48 heures. On siphonne le surnageant et on porte son pH à 7,00 au moyen d'hydroxyde de sodium 5N. On ajoute 19 litres d'éthanol. On laisse reposer 48 heures. On siphonne le surnageant pour le séparer. Le précipité est récupéré, lavé à l'éthanol, broyé et séché sous vide. On obtient ainsi 120 g de mélange d'oligosaccharides non réduit qui constitue le produit OS 4-10-CHO.

**b)** Couplage de la peroxidase de raifort avec le OS 4-10-CHO:

12,5 mg de peroxidase de raifort poudre sont mis en solution dans 3 ml de tampon phosphate 0,05 M, NaCl 0,5 M, pH 7. On ajoute 25 mg de cyanoborohydrure de sodium dans 1 ml du même tampon et 805 mg d'OS 4-10-CHO dans 1 ml du même tampon. On ajuste le pH à 7 avec de la soude 2N et on laisse réagir sous agitation pendant 5 jours à la température de + 4° C. On constate une légère opalescence.

**c)** Elimination du précipité:

On centrifuge à 15.000 rpm pendant 10 minutes, à la température de +4° C, puis on effectue une filtration sur filtre Millipore 0,2 microns.

**d)** Gel filtration:

Le produit récupéré à l'étape précedente est déposé sur une colonne Ultrogel(R) AcA 44 (IBF, France) de dimension 2,6 x 100 cm. L'opération est suivie au spectrophotomètre à 280 nm et on récupère le pic de protéine.

**e)** Caractérisation du produit:

On étudie le taux de couplage du produit en dosant les protéines par la méthode au Bleu de Coomassie (S.M. Read et D.H. Northcote, Ann. Biochem., 1981, 116, 53-64) et le OS 4-10 CHO par la méthode au carbazole(dosage des acides uroniques, R. Bitter et H. Muir, Ann. Biochem., 1962, 4, 330-334).

Les résultats sont calculés pour un PM moyen de 2.000 daltons en ce qui concerne le OS 4-10-CHO et 40.000 daltons en ce qui concerne la peroxidase.

Les résultats sont les suivants:
Protéines : 1,028 mg/ml, soit pour 7 ml: 7,196 mg (0,18 µmoles)
OS 4-10-CHO: 0,152 mg/ml, soit pour 7 ml: 1,064 mg (0,552 µmoles)
Activité : 1.420 u/ml, soit pour 7 ml: 9.940 u
Rendement : 70,6 %.

On obtient 3 moles de OS 4-10-CHO pour 1 mole de peroxydase.

**PREPARATION II**

Préparation du GAG-albumine selon la formule (I) dans laquel P est l'albumine et Z-Oside est le OS 4-10-CHO.

**a)** Génération de la terminaison aldéhydique par dépolymérisation de l'héparine:

On procède comme décrit ci-dessus au paragraphe PREPARATION I a).

**b)** Couplage de l'albumine avec le OS 4-10-CHO:

0,1 ml d'albumine bovine en solution à 30 mg sont mis en solution dans 2,0 ml de tampon phosphate 0,05 M, NaCl 0,5 M, pH 7. On ajoute 60 mg de cyanoborohydrure de sodium dans 1 ml du même tampon et 800 mg de OS 4-10-CHO dans 1 ml du même tampon. On ajuste le pH à 7 avec de la soude 2N et on laisse réagir sous agitation pendant 7 jours. On constate un important insoluble.

**c)** Elimination de l'insoluble:

On centrifuge à 15.000 rpm à la température de +4° C, puis on effectue une filtration sur filtre Millipore 0,2 microns, et on lave avec 1 ml de tampon phosphate.

**d)** Gel filtration:

Le produit récupéré à l'étape précedente est déposé sur une colonne Ultrogel(R) AcA 44 (IBF, France) de dimension 2,6 x 100 cm. L'opération est suivie au spectrophotomètre à 280 nm et on récupère le pic de protéine.

**e)** Caractérisation du produit:

On étudie le taux de couplage du produit en dosant les protéines par la méthode au Bleu de Coomassie et le OS 4-10-CHO par la méthode au carbazol. Les résultats sont calculés pour un PM moyen de 2.000 daltons en ce qui concerne le OS 4-10-CHO et 65.000 daltons en ce qui concerne l'albumine.

Les résultats sont les suivants:

Protéines : 4,6 mg/ml, soit pour 7 ml: 32 mg (0,47 µmoles)

OS 4-10-CHO: 1,22 mg/ml, soit pour 7 ml: 8,54 mg (4,28 µmoles)

On a donc 9 moles de OS 4-10-CHO pour 1 mole d'albumine.

**EXEMPLE**

(a) Adsorption du polycation sur les puits de la plaque de microtitration

250 µl d'une solution de Poly-L-lysine (PM 5000-9000, vendue par Serva, France) à la concentration de 100µg/ml dans un tampon bicarbonate 0,1 M, pH 9,6 sont déposés dans chacun des 96 puits d'une plaque de microtitration. On laisse incuber 48 heures à la température de +4° C.

Les puits sont ensuite vidés par aspiration, puis lavés avec 2 fois 300 µl de soluté chloruré isotonique (NaCl 9 p. 1000). Les plaques sont prêtes pour le dosage.

(b) Incubation du liquide dans lequel se trouve le polyanion sulfaté à doser et préparation de la courbe d'étalonnage

On introduit dans chaque puits 200 µl de la solution échantillon à doser et parallèlement on établit une courbe d'étalonnage en mettant dans un nombre de puits approprié 200 µl du même milieu biologique auquel on aura ajouté une quantité connue du polyanion sulfaté que l'on désire doser, soit 0,0 -0,25 - 0,5 - 1 - 2,5 et 5 µg.

Tous les milieux biologiques ont été additionnés de benzamidine à la concentration de 0,0125 M.

On laisse incuber pendant 2 heures, à la température de +4° C. On lave ensuite avec 2 fois 300 µl de soluté chloruré isotonique.

(c) Mise en contact avec le mélange révélateur

On prépare le mélange révélateur à partir des conjugués GAG-peroxydase et GAG-albumine dans la proportion, exprimée en équivalent de produit pur, de 3,4 µg/ml de peroxydase pour 7,7 µg/ml d'albumine, en solution dans du soluté NaCl 9 p. 1000.

On dépose 200 µl de ce mélange dans chaque puits. On laisse incuber 1 heure à +4° C. On lave ensuite avec 2 fois 400 µl de soluté chloruré isotonique.

(d) Révélation de la quantité d'enzyme présente

On dépose dans chaque puits 100 µl du mélange suivant:
- 0,5 mg d'orthophénylène diamine dans 1 ml de tampon citrate de sodium /HCl 0,1 M, pH 3,5;
- 0,75 µl de peroxyde d'hydrogène.

On laisse incuber 10 minutes à température ambiante (environ 20 à 20° C). On arrête la réaction par addition dans chaque puits de 100 µl d'acide sulfurique 1N. On détermine ensuite la densité optique à 492 nm.

La courbe d'étalonnage est donné en Figure 1. Elle donne en ordonnée la densité optique et en abscisse le log de la concentration exprimée en µg/ml.

On se reporte à cette courbe d'ètalonnage pour déterminer, à partir de la densité optique de l'èchantillon à doser, la concentration en produit de l'èchantillon.

Les produits A) à F) de la liste suivante ont été précédemment introduits dans un mélange de plasma humain et dosés.

Les produits testés sont les suivants:

A) Héparine du commerce, lot H-801 (Laboratoire Choay, Paris, France)

B) Dextrane sulfate, lot 12-837 (Pharmacia, Suède)

C) Fragment d'héparine vendu dans le commerce sous la dénomination Fraxiparine, lot XH-716 (Laboratoire Choay, Paris, France)

D) Pentosane polysulfate vendu dans le commerce sous la dénomination Hémoclar, lot 82003 (Laboratoire Midy, Paris, France)

E) Dosage du fragment d'héparine connu sous la dénomination CY 222 et décrit dans la demande de brevet européen EP 037 319, lot P 54 WH (Laboratoire Choay, Paris, France)

F) Dosage d'un fragment d'héparine obtenu par dépolymérisation au periodate et ayant les caractéristiques suivantes:
PM moyen : 6.000 daltons
Site de fixation à l'/AT III : néant
Structure : essentiellement constitué du disaccharide trisulfaté régulier de type héparinique
Activité anti-Xa : inférieur à 15 u/mg
Activité anti-IIa : inférieur à 15 u/mg
Code : IC 1772 - lot P 37 VH (Laboratoire Choay, Paris, France)

Le produit IC 1772 a été préparé de la manière suivante.

(1) Coupure des chaînes d'héparine à l'aide d'acide periodique

10 g d'héparine injectable de mucus de porc, sous forme de sel de sodium, titrant 157 ul/mg dans le

dosage Codex et 155 u/mg dans le dosage anti-facteur Xa de Yin et al., sont mis en solution dans 250 ml d'eau déminéralisée à 4 °C. Le pH de la solution est ajusté à 5,0 par de l'acide chlorhydrique concentré. 10 g de métaperiodate de sodium ($NaIO_4$, PM: 213,89) en solution dans 250 ml d'eau déminéralisée sont ajoutés sous agitation modérée. Le pH de l'ensemble est ajusté à 5,0 par de l'acide chlorhydrique concentré. La solution est abandonnée 24 heures, à l'obscurité, en chambre froide à 4 °C.

(2) Elimination du periodate residuel

On repartit ensuite la solution réactionnelle dans 3 boyaux de dialyse NOJAX 40^R (porosité de 3 à 4000 Da) et on la soumet à une dialyse 15 heures contre de l'eau déminéralisée courante.

(3) Dépolymérisation en milieu basique

Aux 780 ml de solution obtenue après dialyse, sont ajoutés 16 ml de soude 10N, et l'ensemble est soumis à agitation 3 heures à température ambiante (de l'ordre de 18-21 °C).

(4) Reduction

500 mg de borohydrure de sodium ($NaBH_4$, PM : 37,83) sont alors ajoutés et la solution est agitée de nouveau 4 heures à la température ambiante. Son pH est ensuite amené à 4 à l'aide d'acide chlorhydrique concentré. Après 15 minutes d'agitation, le pH est ajusté à 7 à l'aide de soude concentrée.

Aux 820 ml de solution ainsi obtenue sont ajoutés 16,4 g de NaCl puis 1270 ml d'éthanol.

L'ensemble est laissé au repos 3 heures, puis centrifugé à 2500 t/minute pendant 20 minutes.

Le précipité est recueilli, remis en suspension dans 200 ml d'éthanol pur, broyé à l'Ultra-Turrax ^R et finalement récupéré par filtration sur buchner fritté.

Il est alors séché sous vide à 40 °C pendant 5 heures. On récupère ainsi 8,9 g de produit intermédiaire ayant les propriétés suivantes:
- dosage Codex : 8 ul/mg
- dosage APTT : 7 ul/mg
- dosage Anti-Xa : 8 u/mg.

(5) Fractionnement alcoolique

Ces 8,9 g sont dissous dans environ 120 ml d'eau déminéralisée à température ambiante. On ajoute 1,78 g de NaCl et le pH de la solution est abaissé à 3,5 à l'aide d'acide chlorhydrique. Le volume de la solution est ajusté à 178 ml à l'aide d'eau déminéralisée. 151 ml d'éthanol pur sont ajoutés sous agitation. L'agitation est maintenue 15 minutes après la fin de l'addition puis l'ensemble est laissé au repos 10 heures, à température ambiante.

Le précipité formé est recueilli par centrifugation 20 minutes à 2500 t/min. Il est remis en suspension dans 150 ml d'éthanol pur, broyé à l'Ultra-Turrax, récupéré par filtration sur buchner fritté, lavé par 300 ml d'éthanol pur et finalement séché sous vide à 40 °C pendant 24 heures.

On récupère ainsi sous forme de poudre blanche 5 g du produit IC 1772 ayant les caractéristiques suivantes:
- titre Codex : 11 ul/mg
- titre APTT : 9 ul/mg
- titre Anti-Xa : 12 u/mg.

Les résultats obtenus sont rapportés sur les figures 1 à 6 qui représentent les courbes d'étalonnage obtenues avec dans l'ordre les produits A à F.

Il ressort des figures 1 à 6 qu'en se basant sur la méthode de dosage décrite dans la présente invention, on obtient une variation de la densité optique qui est fonction de la quantité de produit présente dans le plasma. Cette variation de densité optique permet donc d'évaluer dans un échantillon donné, par exemple le plasma d'un animal ou d'un patient traité par l'un des produits (polyanions sulfatés forts) la teneur de ce plasma en produit considéré.

## Revendications

1. Procédé de dosage de polyosides sulfatés fortement anioniques dans un liquide, caractérisé en ce qu'il est basé sur le dosage, grâce à un conjugué anionique révélateur, des sites cationiques restés libres sur une surface recouverte d'un polycation, après que celui-ci ait été mis en contact avec le liquide contenant la polyoside anionique sulfaté à doser, le conjugué anionique révélateur étant choisi de telle manière que sa densité de charge lui permette de se fixer sur les sites restés vacants sur le polycation recouvrant la surface, sans toutefois déplacer le polyoside à doser qui s'y trouve préalablement fixé, et la quantité du produit à doser étant déterminée par référence à une courbe d'étalonnage établie simultanément sur la même plaque.

2. Procédé de dosage de polyosides sulfatés fortement anioniques dans un liquide, caractérisé en ce que:

(a) on met en contact une quantité déterminée du liquide biologique contenant le produit à doser avec une surface recouverte d'un produit cationique;

(b) on met en contact l'ensemble ainsi obtenu avec un excès d'un conjugué anionique révélateur, choisi de telle manière que sa densité de charge lui permette de se fixer sur les sites restés vacants sur le produit cationique de l'étape (a) sans toutefois déplacer le polyoside qui s'y trouve préalablement fixé;

(c) on élimine l'excès du mélange révélateur qui ne s'est pas fixé par liaison ionique avec l'excès du produit cationique de l'étape (a);

(d) on dose, par une méthode appropriée, fonction du conjugué révélateur choisi, la quantité dudit conjugué révélateur qui s'est fixé par liaison ionique au cours de l'étape (b);

(e) on détermine la quantité totale du produit à doser dans l'échantillon en rapportant la quantité du conjugué révélateur définie selon la méthode appropriée de l'étape (d) à une courbe d'étalonnage établie simultanément sur la même plaque selon la même méthode.

3. Procédé selon l'une quelconque des revendications 1 et 2, caractérisé en ce que le polycation utilisé est un polymère basique choisi dans le groupe constitué par le sulfate de protamine, le polybrène, la polyarginine et la poly-L-lysine.

4. Procédé selon la revendication 3, caractérisé en ce que l'on utilise comme polycation une poly-L-lysine d'un poids moléculaire moyen se situant entre 6.000 et 9.000 daltons.

5. Procédé selon l'une quelconque des revendications 2 à 4, caractérisé en ce que dans l'étape (a) le polycation est adsorbé sur des puits d'une plaque de microtitration, en milieu basique, par incubation pendant 24 à 72 heures, de préférence 48 heures à une température se situant entre 0 et 20° C, puis lavage.

6. Procédé selon la revendication 5, caractérisé en ce que le milieu basique dans lequel se trouve le polycation est un tampon tris-phosphate ou un tampon bicarbonate à pH compris entre 9 et 10.

7. Procédé selon la revendication 2, caractérisé en ce que le conjugué anionique révélateur de l'étape (b) est constitué d'un glycosaminoglycane lié de façon covalente à un enzyme.

8. Procédé selon l'une quelconque des revendications 2 à 7, caractérisé en ce que dans l'étape (b) le mélange est incubé au moins 2 heures à une température se situant entre 0 et 20° C, puis lavage.

9. Procédé selon l'une quelconque des revendications 2 à 8, caractérisé en ce que dans l'étape (d) on utilise une méthode de dosage enzymatique.

10. Procédé selon la revendication 9, caractérisé en ce que l'enzyme du conjugué révélateur est dosable par action sur un substrat et libération d'un produit coloré, que l'on dose par densité optique.

11. Procédé selon l'une quelconque des revendications 1 à 10, caractérisé en ce que le polyoside anionique du conjugué révélateur est un glycosaminoglycane.

12. Procédé selon la revendication 11, caractérisé en ce que le glycosaminoglycane du conjugué révélateur est l'héparine ou l'un de ses fragments.

13. Procédé selon la revendication 12, caractérisé en ce que le glycosaminoglycane est un mélange de fragments d'héparine constitués de chaînes possédant essentiellement de 4 à 10 unités disaccharidiques.

14. Procédé selon l'une quelconque des revendications 1 à 13, caractérisé en ce que le conjugué révélateur est un conjugué GAG-enzyme dilué avec un conjugué GAG-albumine, les GAGs utilisés pour les deux conjugués étant identiques.

15. Procédé selon l'une quelconque des revendications 1 à 14, caractérisé en ce que l'enzyme utilisé est la peroxydase de raifort.

16. Procédé selon l'un quelconque des revendications 1 à 15, caractérisé en ce que les constituants de GAG-enzyme sont respectivement le OS 4-10-CHO et la peroxydase de raifort.

17. Procédé selon les revendications 15 et 16, caractérisé en ce que la quantité d'enzyme présente dans chacun des puits est révélée par un mélange d'orthophénylène diamine et de peroxyde d'hydrogène.

18. Procédé selon l'une quelconque des revendications 2, 15, 16 ou 17, caractérisé en ce que dans l'étape (d) on révèle la quantité d'enzyme présente par addition d'un mélange d'orthophénylène diamine en tampon citrate, pH 3,5 et de peroxyde d'hydrogène et incubation 10 minutes à température ambiante, puis arrêt de la réaction par addition d'un acide fort.

19. Procédé selon l'une quelconque des revendications 1 à 14, caractérisé en ce que l'enzyme utilisé est la phosphatase.

20. Procédé selon la revendication 19, caractérisé en ce que le substrat de dosage du conjugué GAG-phosphatase est le 4-nitrophényl phosphate.

21. Coffret destiné au dosage de polyosides sulfatés fortement anioniques caractérisé en ce qu'il contient les éléments suivants:

- Plaque de microtitration
- Polycation
- Conjugué révélateur constitué par un polyanion lié de façon covalente à un révélateur choisi dans le groupe constitué par un enzyme et une molécule organique susceptible d'être marquée
- Substrat de dosage choisi en fonction du conjugué révélateur.

22. Coffret selon la revendication 21, caractérisé en ce qu'il contient les éléments suivants:

- Plaque de microtitration
- Polycation choisi dans le groupe constitué par le sulfate de protamine, le polybrène, la poly-arginine et la poly-L-lysine
- GAG-enzyme lyophilisé
- Substrat de l'enzyme choisi, générateur d'un colorant.

23. Coffret selon la revendication 21 ou 22, caractérisé en ce qu'il contient les éléments suivants:

- Plaque de microtitration
- Polycation choisi dans le groupe constitué par le sulfate de protamine, le polybrène, la poly-arginine et la poly-L-lysine
- GAG-enzyme lyophilisé, l'enzyme étant choisi dans le groupe constitué par la peroxydase de raifort et la phosphatase
- GAG-albumine lyophilisé
- Substrat de l'enzyme choisi, générateur d'un colorant.

24. Coffret selon l'une quelconque des revendications 21 à 23, caractérisé en ce qu'il contient un multiple de chacun des éléments suivants:

- Plaque de microtitration : 1
- Poly-L-lysine : 25 ml à 100 µg/ml
- GAG-peroxydase lyophilisé : 68 µg
- GAG-albumine lyophilisé : 54µg
- Orthophénylène diamine : 10 ml (5 mg).

25. Coffret selon l'une quelconque des revendications 21 à 24, caractérisé en ce que les plaques de microtitration sont recouvertes du polycation et prêtes à l'emploi.

26. Coffret selon l'une quelconque des revendications 21 à 25, accompagné des instructions d'utilisation selon l'une quelconque des revendications 1 à 20.

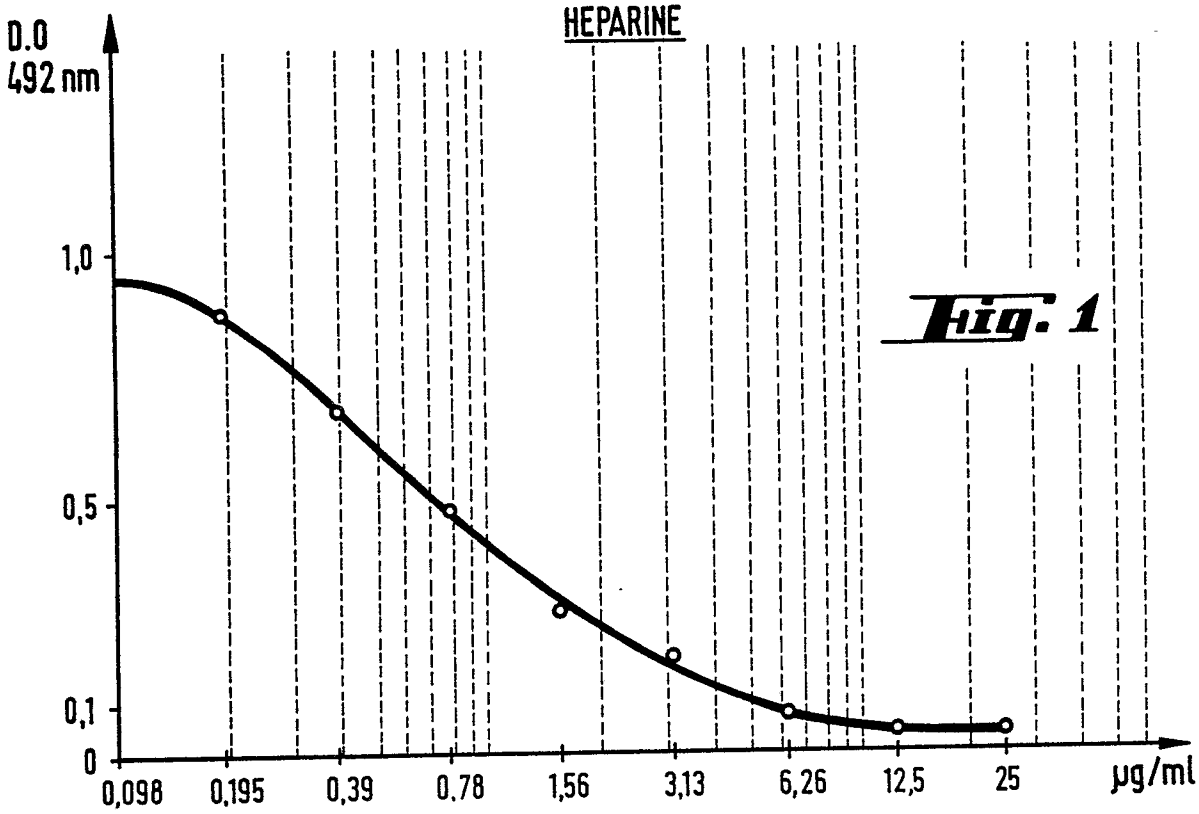
HEPARINE
D.O
492 nm
1,0
0,5
0,1
0
0,098
0,195
0,39
0,78
1,56
3,13
6,26
12,5
25
µg/ml

Fig. 1

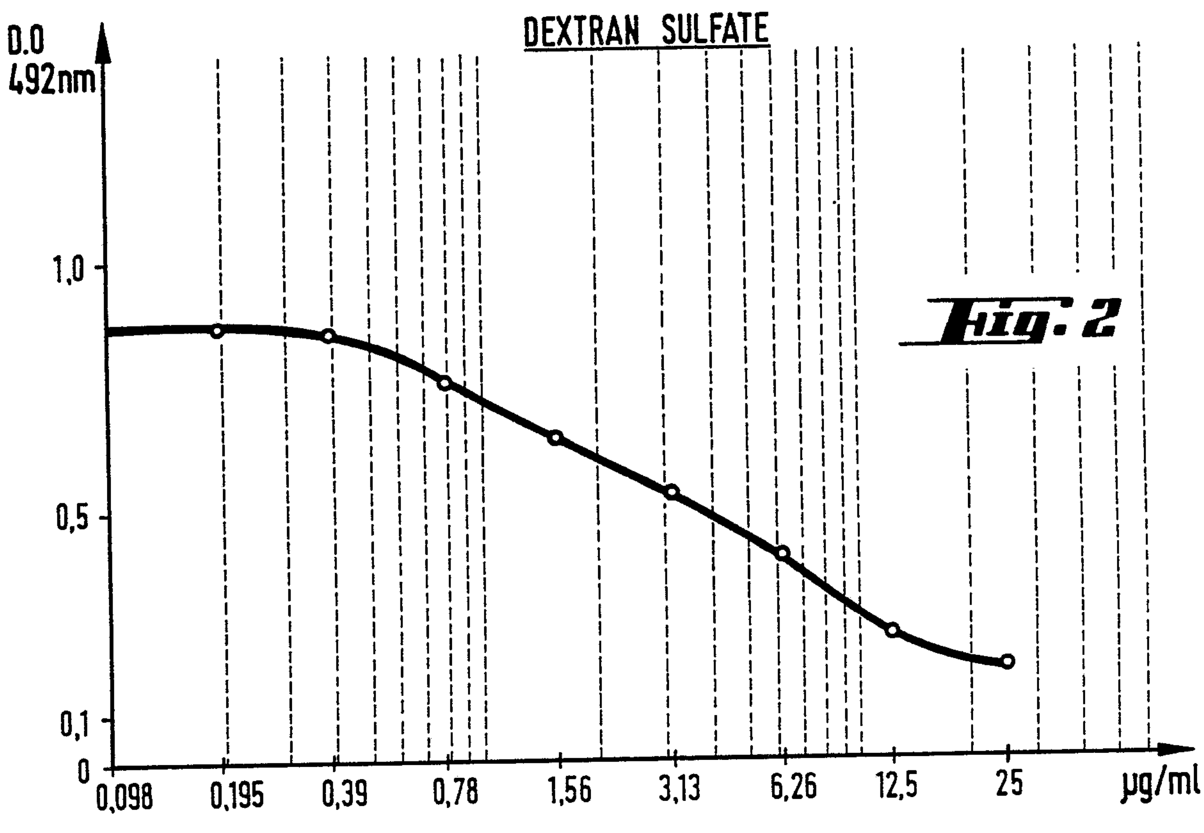
DEXTRAN SULFATE
D.O
492nm
1,0
0,5
0,1
0
0,098
0,195
0,39
0,78
1,56
3,13
6,26
12,5
25
µg/ml

Fig. 2

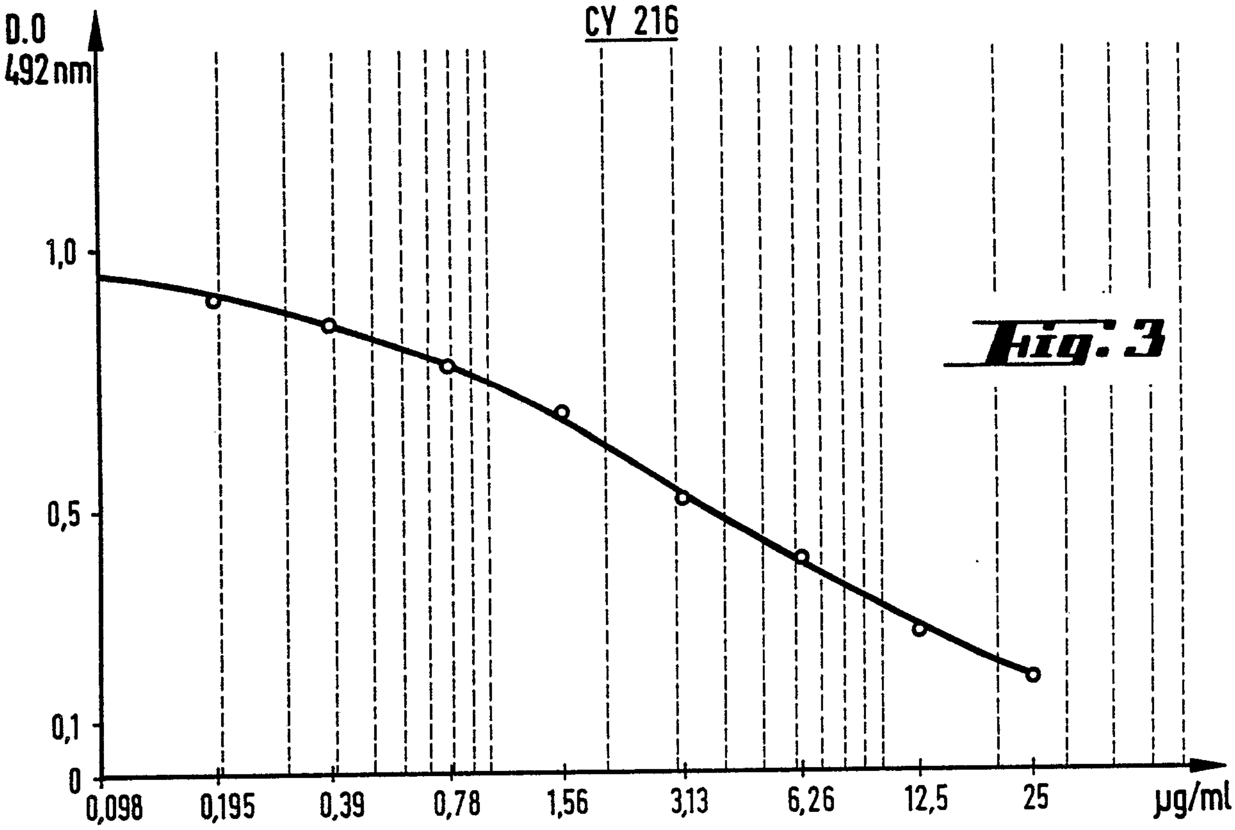
CY 216
D.O
492 nm
1,0
0,5
0,1
0
0,098
0,195
0,39
0,78
1,56
3,13
6,26
12,5
25
µg/ml

Fig. 3

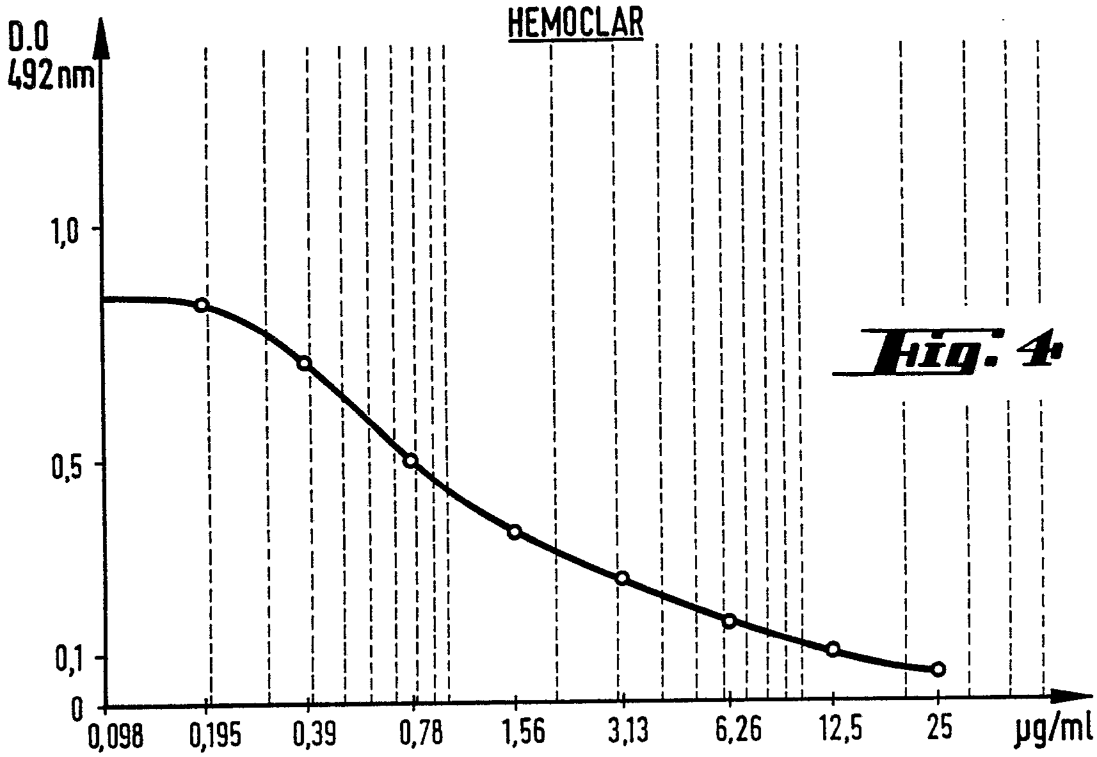
HEMOCLAR
D.O
492 nm
1,0
0,5
0,1
0
0,098
0,195
0,39
0,78
1,56
3,13
6,26
12,5
25
µg/ml

Fig. 4

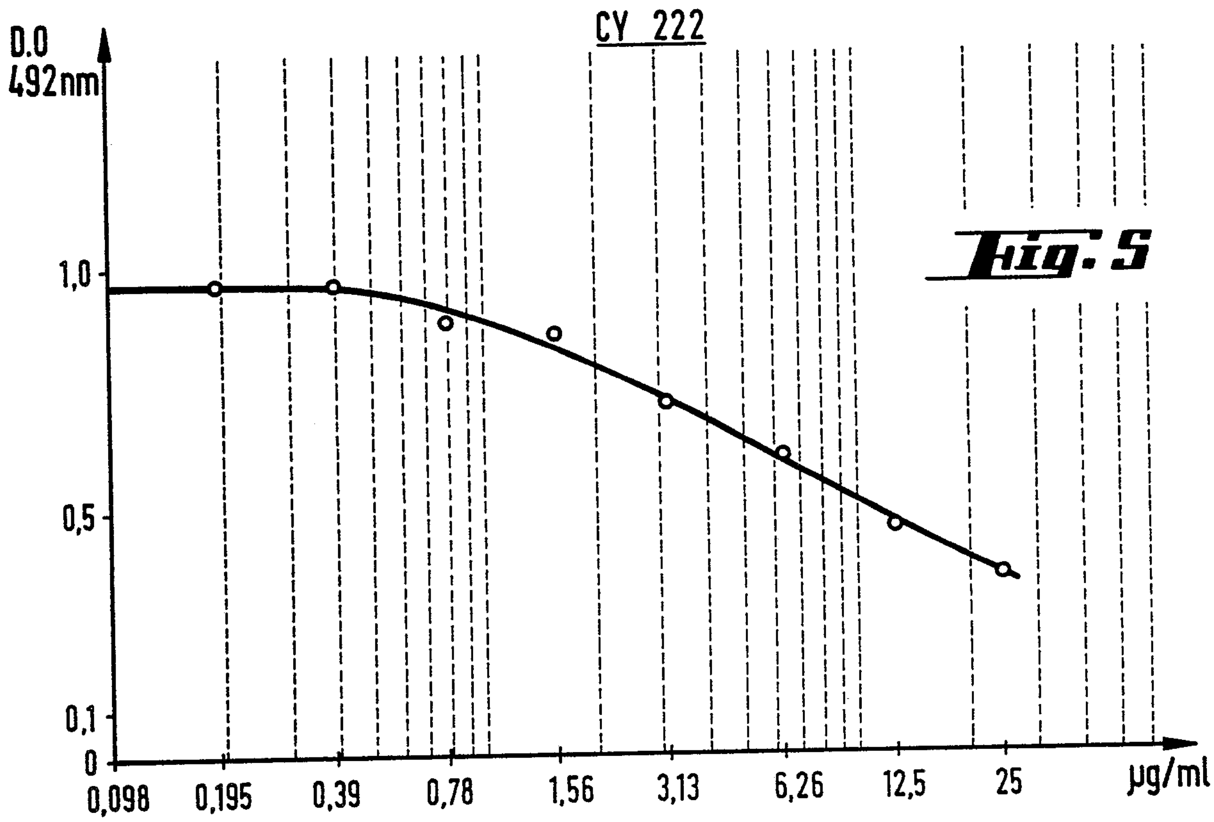
CY 222
D.O
492nm
1,0
0,5
0,1
0
0,098
0,195
0,39
0,78
1,56
3,13
6,26
12,5
25
µg/ml

Fig. 5

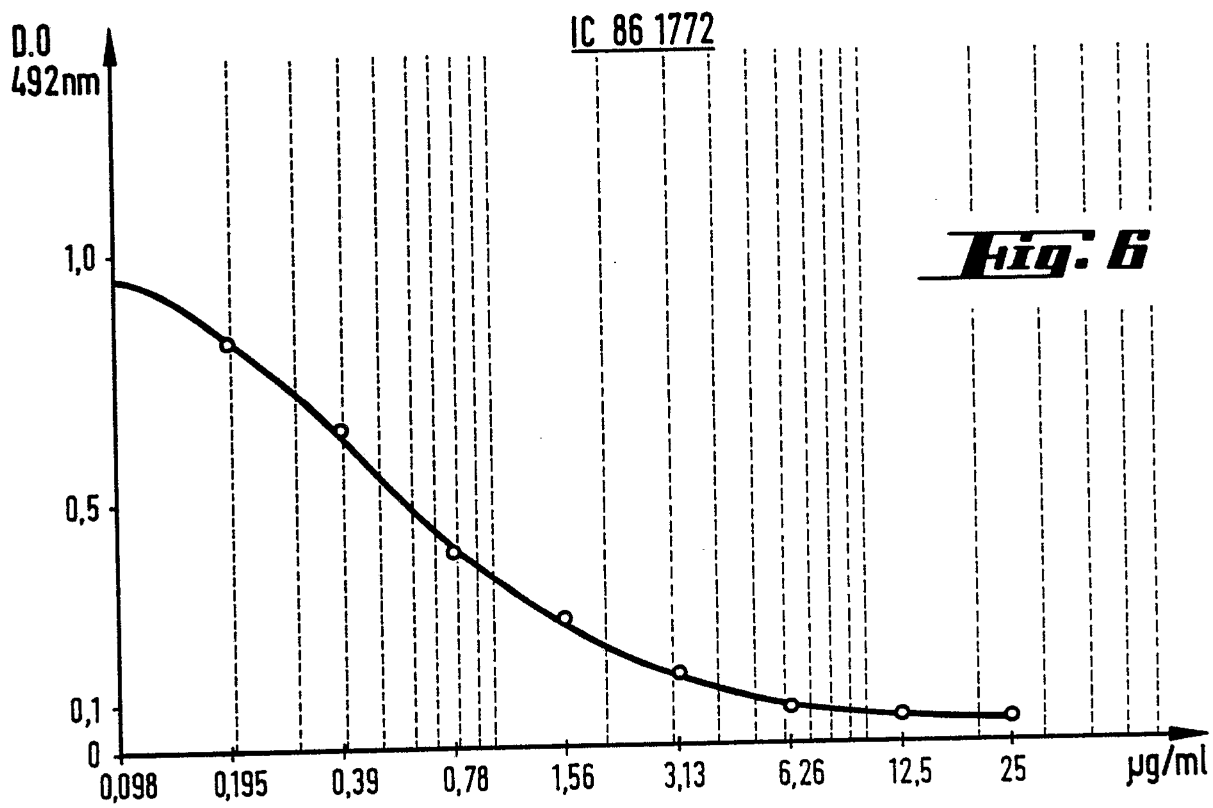
IC 86 1772
D.O
492nm
1,0
0,5
0,1
0
0,098
0,195
0,39
0,78
1,56
3,13
6,26
12,5
25
µg/ml

Fig. 6

Office européen des brevets

# RAPPORT DE RECHERCHE EUROPEENNE

Numero de la demande

EP 89 40 2691

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | THROMBOSIS AND HAEMOSTASIS, vol. 54, no. 3, 1985, pages 630-634, F.K. Schattauer Verlag GmbH, Stuttgart, DE; J. DAWES et al.: "The measurement of heparin and other therapeutic sulphated polysaccharides in plasma, serum and urine" * Pages 630-631 * | 1-3 | G 01 N 33/86 |
| A | WO-A-8 707 183 (NEW YORK UNIVERSITY) * Résumé; pages 1-8 * | 1,2 | |
| A,D | EP-A-0 084 999 (CHOAY S.A.) * Résumé; pages 42-43 * | 4 | |
| | | | **DOMAINES TECHNIQUES RECHERCHES (Int. Cl.5)** |
| | | | G 01 N<br>C 12 Q |

Le présent rapport a été établi pour toutes les revendications

| Lieu de la recherche | Date d'achèvement de la recherche | Examinateur |
|---|---|---|
| LA HAYE | 22-11-1989 | HITCHEN C.E. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)